(19) Europäisches Patentamt
European Patent Office
Office européen des brevets

(11) Numéro de publication: **0 237 438 B1**

(12) **FASCICULE DE BREVET EUROPEEN**

(45) Date de publication du fascicule du brevet: **27.12.90**

(51) Int. Cl.[5]: **C07C 47/21**, C07C 45/51, C07C 43/315, C07C 43/17

(21) Numéro de dépôt: **87400549.9**

(22) Date de dépôt: **12.03.87**

(54) **Procédé de préparation d'aldéhydes polyéniques.**

(30) Priorité: **14.03.86 FR 8603668**

(43) Date de publication de la demande: **16.09.87 Bulletin 87/38**

(45) Mention de la délivrance du brevet: **27.12.90 Bulletin 90/52**

(84) Etats contractants désignés: **AT BE CH DE FR GB IT LI LU NL SE**

(56) Documents cités:
**CH-A- 326 417**
**FR-A- 1 171 938**
**FR-A- 1 438 608**
**FR-A- 2 353 510**
**GB-A- 2 028 330**
**US-A- 2 734 091**

**TETRAHEDRON LETTERS, no. 8, 1978, pages 717-720, Pergamon Press, GB; R.H. WOLLENBERG: "Efficient conversion of carbonyl compounds to conjugated dienals"**

(73) Titulaire: **RHONE-POULENC SANTE, 20, avenue Raymond Aron, F-92160 Antony(FR)**

(72) Inventeur: **Duhamel, Lucette, 32 rue Jacques Boutrolle, F-76130 Mont-Saint-Aignan(FR)**
Inventeur: **Duhamel, Pierre, 32 rue Jacques Boutrolle, F-76130 Mont-Saint-Aignan(FR)**
Inventeur: **Lecouve, Jean-Pierre, 23 E rue de l'Oratoire, F-69300 Caluire(FR)**

(74) Mandataire: **Le Pennec, Magali et al, RHONE-POULENC SANTE, Service Brevets, 20 Avenue Raymond Aron, F-92165 Antony Cédex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'aldéhydes polyéniques de formule générale :

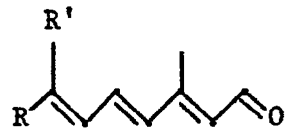

(I)

dans laquelle R représente un radical hydrocarboné et R′ représente un atome d'hydrogène ou un radical alkyle contenant 1 à 4 atomes de carbone.

D'un intérêt tout particulier sont les produits de formule générale (I) dans laquelle R′ représente un groupement méthyle et R représente un radical méthyle (déhydrocitral), diméthyl-4,8 nonatriène-1,3,7 yle (pseudo-rétinal) ou (triméthyl-2,6,6 cyclohexène-1 yl)-2 éthényle (rétinal).

Selon la présente invention les produits de formule générale (I) peuvent être obtenus par action d'un composé carbonylé de formule générale :

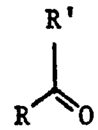

(II)

dans laquelle R et R′ sont définis comme précédemment sur un dérivé métallique préparé in situ par échange métal-halogène avec un composé halogéné de formule générale :

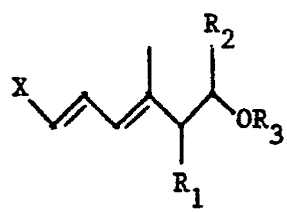

(III)

dans laquelle X représente un atome d'halogène (de préférence un atome de brome), et $R_1$ représentant un atome d'hydrogène, $R_2$ représente un radical alcoxy identique à $OR_3$, $R_3$ représentant un radical alkyle contenant 1 à 4 atomes de carbone (de préférence méthyle ou éthyle), ou bien $R_1$ et $R_2$ formant ensemble une liaison, $R_3$ représente un radical alkyle contenant 1 à 4 atomes de carbone (de préférence méthyle ou éthyle) suivie de l'hydrolyse du produit obtenu.

Les dérivés métalliques du produit de formule générale (III) qui conviennent pour la réalisation de la présente invention sont les dérivés du lithium, du magnésium et du cuivre. D'un intérêt tout particulier sont les dérivés du lithium.

Les dérivés métalliques du produit de formule générale (III) sont obtenus, in situ, par action d'un dérivé organo-métalliques sur un produit de formule générale (III) en opérant dans un solvant organique inerte anhydre tel que l'éther diéthylique ou le tétrahydrofuranne à une température inférieure à –50°C et de préférence voisine de –70°C. Comme dérivé organo-métallique, il est particulièrement avantageux d'utiliser le tertiobutyllithium.

La condensation d'un composé carbonylé de formule générale (II) sur le dérivé métallique du produit de formule générale (III) préparé in situ, s'effectue dans le même solvant à une température inférieure à 0°C et généralement à une température inférieure à –30°C.

Selon la signification de $R_1$, $R_2$, $R_3$, la conversion par deshydratation et hydrolyse du produit obtenu est effectuée au moyen d'un acide minéral (acide chlorhydrique) en milieu hydro-organique (tétrahydrofuranne-eau ; éther éthylique-eau) à une température comprise entre –60°C et +30°C ($R_1$ et $R_2$ représentant une liaison et $OR_3$ représente un radical alcoxy) ou bien au moyen d'un acide minéral (acide bromhydrique) en milieu hydro-organique (acétone-eau) à une température comprise entre 0°C et la température d'ébullition du mélange réactionnel et, plus particulièrement, dans les conditions décrites dans le brevet français FR 7 824 350 (2 434 135) ($R_1$ représente un atome d'hydrogène et $R_2$ représente un radical alcoxy identique à $OR_3$).

Le produit de formule générale (III) dans laquelle $R_1$ représente un atome d'hydrogène et $R_2$ représente un radical alcoxy identique à $OR_3$, $R_3$ représentant un radical alkyle contenant 1 à 4 atomes de carbone, peut être obtenu par action d'un halogénure phosphonium, tel que le bromure de bromométhyltriphénylphosphonium, sur un dialcoxy-5,5 méthyl-3 pentène-2 al en opérant en présence d'un alcoolate de métal alcalin, tel que le tertiobutylate de potassium, dans un solvant organique anhydre, tel que le tétrahydrofuranne, à une température comprise entre −70°C et 0°C.

Le dialcoxy-5,5 méthyl-3 pentène-2 al peut être préparé selon le procédé décrit dans le brevet français FR 7 715 070 (2, 391 181).

Le produit de formule générale (III) dans laquelle $R_1$ et $R_2$ forment une liaison et $R_3$ représente un radical alkyle contenant 1 à 4 atomes de carbone peut être obtenu par action de l'iodure de triméthylsilyl en présence d'hexaméthyldisilazane sur un produit de formule générale (III) dans laquelle $R_1$ représente un atome d'hydrogène et $R_2$ représente un radical $OR_3$ selon la méthode décrite par R.D. Miller et D.R. McKean, Tetrahedron Letters, 23, 323 (1982).

Les produits de formule générale (III) dans laquelle $R_1$ et $R_2$ forment une liaison at $R_3$ représente un radical alkyle contenant 1 à 4 atomes de carbone des produits nouveaux qui constituent un autre objet de la présente invention.

Les exemples suivants, donnés à titre non limitatif, illustrent la présente invention.

EXEMPLE 1

Dans un ballon tricol de 25 cm3 muni d'un thermomètre, d'une agitation magnétique et d'un septum, on introduit, sous atmosphère d'argon, 0,56 g de bromo-6 méthyl-3 diméthoxy-1,1 hexadiène-3,5 (2,38 m.moles) en solution dans 10 cm3 d'éther anhydre. On refroidit à −70°C puis on ajoute en 5 minutes, 2,4 cm3 de tertiobutyllithium 1,8N dans le pentane, soit 1,8 équivalent, en maintenant la température inférieure à −65°C. on maintient pendant 70 minutes à −70°C puis on ajoute 0,33 g de β-ionone (0,72 équivalent) en solution dans 4 cm3 d'éther. On laisse la température remonter à 0°C en 15 minutes puis on maintient à cette température pendant 1 heure 15 minutes. On ajoute alors 3 cm3 d'une solution aqueuse de bicarbonate de sodium à 5 %. Après 20 minutes d'agitation à 0°C, la phase aqueuse est extraite à l'éther et la phase éthérée est lavée à l'eau jusqu'à neutralité puis séchée sur carbonate de sodium. Après filtration et évaporation des solvants, on obtient 0,8 g de produit brut qui est purifié par flash chromatographie sur silice en éluant avec un mélange éther de pétrole éther(85-15 en volumes).

On obtient ainsi 0,45 g de (triméthyl-2,6,6 cyclohexène-1 yl)-9 diméthoxy-1,1 diméthyl-3,7 hydroxy-7 nonatriène-3,5,8 ou hydroxyacétal $C_{20}$ diméthylique.

La structure du produit obtenu est confirmée par le spectre infra-rouge et le spectre de résonance magnétique nucléaire du proton.

Dans un ballon tricol de 100 cm3 muni d'un réfrigérant d'un septum et d'une agitation magnétique, on introduit :

- 0,9 g d'hydroxyacétal $C_{20}$ diméthylique (2,6 m.moles),
- 60 cm3 d'une solution de 192 cm3 d'acétone et de 1 cm3 d'eau,
- 0,02 g de ionol et
- 0,2 cm3 d'eau.

On chauffe au reflux (54°C) pendant 5 minutes puis on ajoute 0,4 cm3 d'une solution de 141 cm3 d'acétone et de 3 cm3 d'acide bromhydrique aqueux à 48 %.

On chauffe pendant 17 minutes au reflux puis on ajoute rapidement 80 cm3 d'eau. On agite pendant 10 minutes puis on extrait au pentane. On lave la phase organique par une solution de bicarbonate de sodium à 5 % puis à l'eau jusqu'à un pH voisin de 7-8 puis on la sèche sur carbonate de sodium. Après filtration et évaporation du solvant, le produit brut obtenu est purifié par chromatographie sur silice en éluant avec un mélange éther de pétrole-éther (98-2 en volumes). On obtient ainsi 0,534 g de rétinal. La rendement de l'hydrolyse est de 72,7 %.

Le bromo-6 méthyl-3 diméthoxy-1,1 hexadiène-3,5 peut être préparé de la manière suivante :

Dans un ballon tricol de 500 cm3, muni d'une agitation magnétique et d'un thermomètre, on introduit, sous atmosphère d'argon, 12,24 g de bromure de bromométhyltriphénylphosphonium (32,8 m.moles) en suspension dans 160 cm3 de tétrahydrofuranne anhydre. On refroidit à −70°C puis on ajoute, en 10 minutes, par petites fractions, 3,15 g de tertiobutylate de potassium (32,8 m.moles). On agite pendant 1 heure 30 minutes à −70°C.

La suspension blanche devient orangée. On ajoute alors, en 10 minutes à −70°C, 3,3 g de diméthoxy-5,5 méthyl-3 pentène-2 al (20,9 m.moles soit 0,75 équivalent) en solution dans 17 cm3 de tétrahydrofuranne. Le mélange réactionnel est maintenu pendant 1 heure à 0°C puis pendant 1 heure 30 minutes à une température voisine de 20°C.

On ajoute rapidement 85 cm3 d'eau puis on agite vigoureusement pendant 10 minutes. On reprend le mélange réactionnel par 100 cm3 d'éther. Après décantation la phase aqueuse est extraite par 6 fois 40 cm3 d'éther. Les phases organiques sont séchées sur sulfate de magnésium. Aprés filtration et évaporation des solvents, on obtient une huile épaisse à laquelle on ajoute 10 g de sable. Après filtration sur silice en éluant avec du pentane on obtient 4,13 g de bromo-6 méthyl-3 diméthoxy-1,1 hexadiène-3,5.

Le rendement est de 84 %.

La structure du produit obtenu est confirmée par le spectre infra-rouge et le spectre de résonance magnétique nucléaire du proton.

EXEMPLE 2

On opère comme dans l'exemple 1 mais utilisant 0,56 g de bromo-6 méthyl-3 diéthoxy-1,1 hexadiène-3,5 (2,12 m.moles) et 0,33 g de β-ionone (1,716 m.mole soit 0,8 équivalent). On obtient, après flash chromatographie, 0,47 g de (triméthyl-2,6,6 cyclohexène-1 yl)-9 diéthoxy-1,1 diméthyl-3,7 hydroxy-7 nonatriène-3,5,8 ou hydroxy acétal $C_{20}$ diéthylique.

Le rendement est de 73 %.

La structure du produit obtenu est confirmée par le spectre infra-rouge et le spectre de résonance magnétique nucléaire du proton.

Dans un ballon tricol de 50 cm3, muni d'un réfrigérant, d'un septum et d'une agitation magnétique, on introduit 30 cm3 d'une solution de 192 cm3 d'acétone et de 1 cm3 d'eau, 0,46 g d'hydroxyacétal $C_{20}$ diéthylique (1,22 m.mole), 0,01 g de ionol et 0,1 cm3 d'eau.

On chauffe au reflux (54°C) pendant 5 minutes, puis on ajoute 0,2 cm3 d'une solution de 141 cm3 d'acétone et de 3 cm3 d'acide bromhydrique aqueux à 48 %. On laisse pendant 17 minutes au reflux puis on ajoute rapidement 40 cm3 d'eau. On agite pendant 10 minutes puis on extrait au pentane. La phase organique est lavée par une solution de bicarbonate de sodium à 5 % puis à l'eau jusqu'à un pH de 7-8 et enfin séchée sur carbonate de sodium. Après filtration et évaporation du solvant, le produit brut est chromatographié sur silice en éluant avec un mélange éther de pétrole-éther (98-2 en volumes). On obtient ainsi 0,25 g de rétinal.

Le rendement est de 72 %.

Le bromo-6 méthyl-3 diéthoxy-1,1 hexadiène-3,5 est préparé dans les conditions décrites précédemment pour l'obtention du bromo-6 méthyl-3 diméthoxy-1,1 hexadiène-3,5. Ainsi, à partir de 4,67 g de diéthoxy-5,5 méthyl-3 penténe-2 al (25,1 m.moles), on obtient, avec un rendement de 77,5 %, 5,1 g de bromo-6 méthyl-3 diéthoxy-1,1 hexadiène-3,5.

La structure du produit obtenu est confirmée par le spectre infra-rouge et le spectre de résonance magnétique nucléaire du proton.

EXEMPLE 3

En opérant comme dans l'exemple 1 mais à partir de 0,43 g de bromo-6 méthyl-3 diéthoxy-1,1 hexadiène-3,5 (1,63 m.mole) et d'un excès d'acétone (0,4 g soit 4 équivalents), on obtient 0,4 g de diéthoxy-1,1 diméthyl-3,7 hydroxy-7 octadiène-3,5 ou hydroxyacétal $C_{10}$ diéthylique qui, après deshydratation et hydrolyse, puis purification par flash chromatographie en éluant avec un mélange éther de pétrole-éther (96-4 en volumes), fournit 0,121 g de déhydrocitral.

Le rendement est de 49,6 % par rapport au bromo-6 méthyl-3 diéthoxy-1,1 hexadiène-3,5 mis en oeuvre.

La structure du produit obtenu est confirmée par le spectre infra-rouge et le spectre de résonance magnétique nucléaire du proton.

EXEMPLE 4

On opère comme dans l'exemple 3 mais à partir de 0,56 g de bromo-6 méthyl-3 diméthoxy-1,1 hexadiène-3,5 (2,38 m.moles) et d'un excès d'acétone (0,4 g soit 4 équivalents). On obtient, après flash chromatographie en éluant avec un mélange éther de pétrole-éther (70-30 en volumes), 0,44 g de diméthoxy-1,1 diméthyl-3,7 hydroxy-7 octadiène-3,5 ou hydroxyacétal $C_{10}$ diméthylique.

Le rendement est de 86,8 %.

L'hydroxyacétal $C_{10}$ diméthylique ainsi obtenu fournit, avec un rendement de 60,2 %, 0,21 g de déhydrocitral identique à celui obtenu à l'exemple 3.

EXEMPLE 5

On opère comme dans l'exemple 1 mais à partir de 0,56 g de bromo-6 méthyl-3 diméthoxy-1,1 hexadiène-3,5 (2,38 m.moles) et de 0,37 g de pseudo-ionone (1,90 m.mole soit 0,8 équivalent). On obtient ainsi, après flash chromatographie, en éluant avec un mélange éther de pétrole-éther (80-20 en volumes), 0,60 g de diméthoxy-1,1 tétraméthyl-3,7,11,15 hydroxy-7 hexadécapentaène-3,5,8,10,14 ou hydroxyacétal $C_{20}$ diméthylique.

Le rendement est de 87,8 %.

L'hydroxyacétal diméthylique ainsi obtenu, fournit avec un rendement de 60,8 %, 0,33 g de tétraméthyl-3,7,11,15 hexadécahexaène-2,4,6,8,10,14 al ou pseudo-rétinal.

La structure du produit obtenu est confirmée par le spectre infra-rouge et le spectre de résonance magnétique nucléaire du proton.

EXEMPLE 6

Dans un ballon de 25 cm3 muni d'un thermomètre et d'une agitation magnétique, on introduit, sous atmosphère d'argon, 0,257 g de bromo-6 méthyl-3 méthoxy-1 hexatriène-1,3,5 (1,26 m.mole) en solution dans 10 cm3 d'éther. On refroidit à –70°C. On ajoute alors en 7 minutes, 1,2 cm3 de tertiobutyllithium 1,8N dans le pentane (2,1 m.moles soit 1,7 équivalent) en maintenant la température inférieure à –68°C. La solution se colore légèrement de jaune pâle à marron clair. On agite pendant 90 minutes à –70 °C. On ajoute ensuite 0,23 g de β-ionone (1,196 m.mole soit 0,95 équivalent) en solution dans 3 cm3 d'éther. On agite pendant 90 minutes à –30°C. On ajoute 4,5 cm3 d'acide chlorhydrique 1N à –60°C puis on agite vigoureusement pendant 1 heure à 10°C. Après décantation, extraction à l'éther et évaporation du solvant on obtient 0,44 g d'un produit brut qui est purifié par flash chromatographie sur silice, en éluant avec un mélange éther de pétrole-éther (98-2 en volumes). On obtient ainsi, avec un rendement de 55 %, 0,187 g de rétinal.

La structure du produit obtenu est confirmée par le spectre infra-rouge et le spectre de résonance magnétique nucléaire du proton.

Le bromo-6 méthyl-3 méthoxy-1 hexatriène-1,3,5 peut être préparé de la manière suivante :

Dans un ballon de 25 cm3, on introduit, sous atmosphère d'argon, 1,17 g de bromo-6 méthyl-3 diméthoxy-1,1 hexadiène-3,5 (4,97 m.moles) en solution dans 10 cm3 de tétrachlorure de carbone. On refroidit à –10°C puis on ajoute 1,31 cm3 d'hexaméthyldisilazane puis 0,81 cm3 d'iodure de triméthysilyle. On laisse pendant 2 heures à 0°C puis laisse monter la température au voisinage de 20°C et maintient ainsi pendant 12 heures. Le mélange réactionnel est repris par 50 cm3 de pentane, filtré puis lavé au pentane. La phase pentanique est lavée par 4 fois 3 cm3 d'une solution saturée de carbonate de sodium. La solution brune devient jaune doré. La phase organique est séchée sur carbonate de sodium pendant 2 heures à 8°C. Après filtration rapide, le solvant est évaporé sous pression réduite (15 mm de mercure ; 2 kPa) en agitant et en présence de carbonate de sodium. Lorsqu'il ne reste plus que 2 cm3 de solution, on distille le bromo-6 méthyl-3 méthoxy-1 hexatriène=1,3,5 sur carbonate de sodium. On obtient ainsi, avec un rendement de 59,1 %, 0,597 g de bromo-6 méthyl-3 méthoxy-1 hexatriène-1,3,5 ($PE_{0,022\ kPa}$ = 80°C).

EXEMPLE 7

Dans un ballon de 25 cm3 muni d'un thermomètre et d'une agitation magnétique, on introduit, sous atmosphère d'argon, 0,5 g de bromo-6 méthyl-3 diméthoxy-1,1 hexadiène-3,5 (2,12 m.moles) en solution dans 10 cm3 de tétrahydrofuranne anhydre. On refroidit à –70°C puis on ajoute, en 7 minutes, 2,1 cm3 de tertiobutyllithium 1,8N dans le pentane (soit 1,8 équivalent) en maintenant la température inférieure à –65°C. On maintient pendant 70 minutes à –70°C. On ajoute ensuite 0,186 g de benzaldéhyde (0,8 équivalent) en solution dans 4 cm3 de tétrahydrofuranne. On maintient à une température voisine de 20°C pendant 40 minutes. On ajoute alors 3 cm3 d'une solution aqueuse de bicarbonate de sodium à 5 %. Après 1 heure d'agitation à une température voisine de 20°C, le mélange réactionnel est repris par 50 cm3 d'éther. Après décantation, la phase aqueuse est extraite à l'éther. Les phases organiques réunies sont lavées à l'eau jusqu'à neutralité puis séchées sur carbonate de sodium. Après filtration et évaporation des solvants, on obtient 0,5 g de diméthoxy-1,1 méthyl-3 phényl-7 hydroxy-7 heptadiène-3,5.

La rendement est quantitatif.

En effectuant la deshydratation et l'hydrolyse de l'hydroxyacétal obtenu dans les conditions habituelles, on obtient le méthyl-3 phényl-7 heptatriène-2,4,6 al.

**Revendications**

1 - Procédé de préparation d'aldéhydes polyéniques de formule générale :

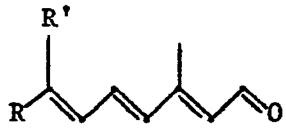

dans laquelle R représente un radical hydrocarboné et R' représente un atome d'hydrogène ou un radical alkyle contenant 1 à 4 atomes de carbone, par deshydratation et hydrolyse simultanée d'un hydroxyacétal ou d'un éther d'énol hydroxylé, caractérisé en ce que l'on obtient un dérivé métallique de l'hydroxyacétal ou de l'éther d'énol hydroxylé par réaction d'un composé carbonylé de formule générale :

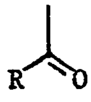

dans laquelle R et R' sont définis comme précédemment sur un dérivé métallique préparé in situ, par échange halogène-métal avec un composé halogéné de formule générale :

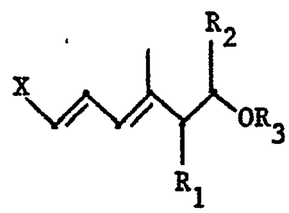

dans laquelle X représente un atome d'halogène, et $R_1$ représentant un atome d'hydrogène, $R_2$ représente un radical $OR_3$, les radicaux $R_3$ étant des radicaux alkyles contenant 1 à 4 atomes de carbone, ou bien $R_1$ et $R_2$ formant ensemble une liaison, $R_3$ représente un radical alkyle contenant 1 à 4 atomes de carbone, et en ce que le dérivé métallique est transformé en hydroxy-acétal ou en éther d'enol hydroxylé.

2 - Procédé selon la revendication 1 caractérisé en ce que le dérivé métallique préparé in situ, est un dérivé du lithium, du magnésium ou du cuivre.

3 - Procédé selon la revendication 1 caractérisé en ce que le dérivé métallique préparé in situ, est un dérivé du lithium.

4 - Procédé selon la revendication 1 caractérisé en ce que le dérivé métallique préparé in situ, est obtenu par action d'un dérivé organo-métallique sur un composé halogéné de formule générale :

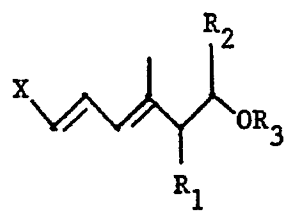

dans laquelle X, $R_1$, $R_2$, et $R_3$ sont définis comme dans la revendication 1 en opérant dans un solvant organique inerte anhydre à une température inférieure à –50°C.

5 - Procédé selon la revendication 4 caractérisé en ce que le dérivé organo-métallique est le tertiobutyllithium et le composé halogéné est le dérivé bromé.

6 - Procédé selon la revendication 1 caractérisé en ce que l'on condense un composé carbonylé de formule générale :

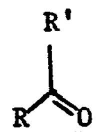

dans laquelle R et R' sont définis comme dans la revendication 1 sur le dérivé métallique préparé in situ à une température inférieure à 0°C en opérant dans un solvant organique inerte anhydre.

7 - Procédé selon l'une des revendications 4 ou 6 caractérisé en ce que le solvant organique inerte anhydre est choisi parmi l'éther diéthylique et le tétrahydrofuranne.

8 - Un produit de formule générale :

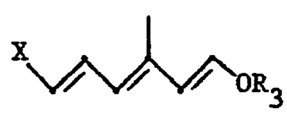

dans laquelle X représente un atome d'halogène et $R_3$ représente un radical alkyle contenant 1 à 4 atomes de carbone.

9 - Le bromo-6 méthyl-3 méthoxyl-1 hexatriène-1,3,5.

**Claims**

1. Process for the preparation of polyene aldehydes of general formula:

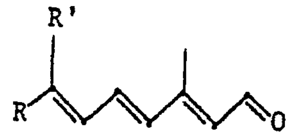

in which R denotes a hydrocarbon radical and R′ denotes a hydrogen atom or an alkyl radical containing 1 to 4 carbon atoms, by dehydration and simultaneous hydrolysis of a hydroxyacetal or of a hydroxylated enol ether, characterized in that a metal derivative of the hydroxyacetal or of the hydroxylated enol ether is obtained by reaction of a carbonyl compound of general formula:

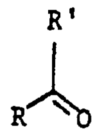

in which R and R′ are defined as above, with a metal derivative prepared in situ by halogen-metal exchange with a halogen compound of general formula:

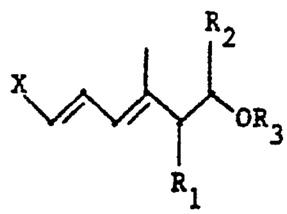

in which X denotes a halogen atom and, with $R_1$ denoting a hydrogen atom, $R_2$ denotes a radical $OR_3$, the radicals $R_3$ being alkyl radicals containing 1 to 4 carbon atoms, or, with $R_1$ and $R_2$ together forming a bond, $R_3$ denotes an alkyl radical containing 1 to 4 carbon atoms, and in that the metal derivative is converted to a hydroxyacetal or a hydroxylated enol ether.

2. Process according to claim 1, characterized in that the metal derivative prepared in situ is a lithium, magnesium or copper derivative.

3. Process according to claim 1, characterized in that the metal derivative prepared in situ is a lithium derivative.

4. Process according to claim 1, characterized in that the metal derivative prepared in situ is obtained by reaction of an organometallic derivative with a halogen compound of general formula:

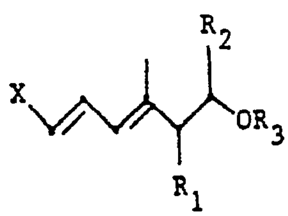

in which X, $R_1$, $R_2$ and $R_3$ are defined as in claim 1, the operation being carried out in an anhydrous inert organic solvent at a temperature below –50°C.

5. Process according to claim 4, characterized in that the organometallic derivative is tert-butyllithium and the halogen compound is the bromo derivative.

6. Process according to claim 1, characterized in that a carbonyl compound of general formula:

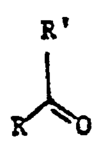

in which R and $R_1$ are defined as in claim 1, is condensed with the metal derivative prepared in situ at a temperature below 0°C, the operation being carried out in an anhydrous inert organic solvent.

7. Process according to either of claims 4 and 6, characterized in that the anhydrous inert organic solvent is chosen from diethyl ether and tetrahydrofuran.

8. Product of general formula:

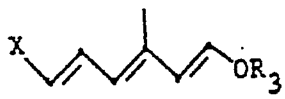

in which X denotes a halogen atom and $R_3$ denotes an alkyl radical containing 1 to 4 carbon atoms.

9. 6-Bromo-3-methyl-1-methoxy-1,3,5-hexatriene.

## Patentansprüche

1. Verfahren zur Herstellung von Polyenaldehyden der allgemeinen Formel:

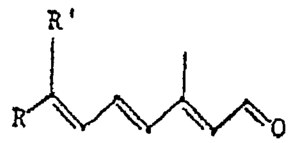

in welcher R einen Kohlenwasserstoffrest darstellt und R′ ein Wasserstoffatom oder einen Alkylrest mit 1 bis 4 Kohlenstoffatomen darstellt, durch Dehydratation und gleichzeitige Hydrolyse eines Hydroxyacetals oder eines hydroxyhaltigen Enoläthers, dadurch gekennzeichnet, daß man ein Metallderivat des Hydroxyacetals oder des hydroxyhaltigen Enoläthers erhält durch Reaktion einer Carbonylverbindung der allgemeinen Formel:

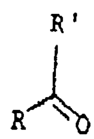

in welcher R und R′ die obige Bedeutung haben, mit einer in situ durch Halogen/Metallaustausch mit einer Halogenverbindung der allgemeinen Formel:

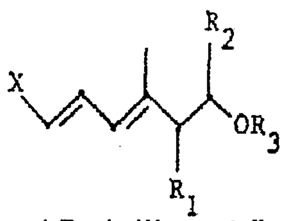

in welcher X ein Halogenatom darstellt und $R_1$ ein Wasserstoffatom darstellt, $R_2$ einen Rest $OR_3$ darstellt, wobei die Reste $R_3$ Alkylreste mit 1 bis 4 Kohlenstoffatomen sind, oder $R_1$ und $R_2$ zusammen eine Bindung bilden und $R_3$ einen Alkylrest mit 1 bis 4 Kohlenstoffatomen darstellt, hergestellten Metallverbindung, und daß man das Metallderivat in den Hydroxy-acetal oder hydroxyhaltigen Enoläther überführt.

2. Verfahren nach Anspruch 1, dadurch gekennzeichnet, daß die in situ hergestellte Metallverbindung eine Lithium-, Magnesium- oder Kupferverbindung ist.

3. Verfahren nach Anspruch 1, dadurch gekennzeichnet, daß die in situ hergestellte Metallverbindung eine Lithiumverbindung ist.

4. Verfahren nach Anspruch 1, dadurch gekennzeichnet, daß die in situ hergestellte Metallverbindung erhalten wird durch Umsetzung einer Organometallverbindung mit einer Halogenverbindung der allgemeinen Formel:

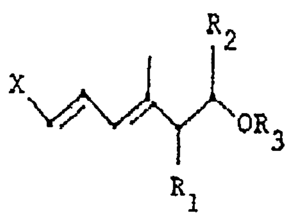

in welcher X, $R_1$, $R_2$ und $R_3$ die im Anspruch 1 angegebene Bedeutung haben, indem man in einem wasserfreien, inerten organischen Lösungsmittel bei einer Temperatur unterhalb –50°C arbeitet.

5. Verfahren nach Anspruch 4, dadurch gekennzeichnet, daß die Organometallverbindung tert. Butyllithium und die Halogenverbindung das Bromderivat ist.

6. Verfahren nach Anspruch 1, dadurch gekennzeichnet, daß man eine Carbonylverbindung der allgemeinen Formel:

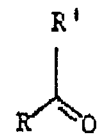

in welcher R und R′ die im Anspruch 1 angegebene Bedeutung haben, mit der in situ hergestellten Metallverbindung bei einer Temperatur unterhalb 0°C kondensiert, indem man in einem wasserfreien, inerten organischen Lösungsmittel arbeitet.

7. Verfahren nach einem der Ansprüche 4 oder 6, dadurch gekennzeichnet, daß das wasserfreie, inerte organische Lösungsmittel ausgewählt ist aus Diäthyläther und Tetrahydrofuran.

8. Verbindung der allgemeinen Formel:

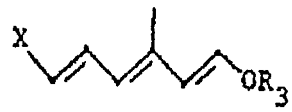

in welcher X ein Halogenatom darstellt und $R_3$ einen Alkylrest mit 1 bis 4 Kohlenstoffatomen darstellt.

9. 6-Brom-3-methyl-1-methoxy-1,3,5-hexatrien.